# EUROPEAN PATENT APPLICATION

(11) **EP 4 574 069 A1**
(43) Date of publication of application: **25.06.2025**
(21) Application number: 24213837.8
(22) Date of filing: 19.11.2024
(51) Int. Cl.: A61B 18/02, F25D 3/10, A61F 7/00

(54) **APPARATUSES AND METHODS FOR TARGETED VAPORIZATION OF CRYOGEN**

(30) Priority: 22.12.2023 US 202318393789
(71) Applicant: Varian Medical Systems, Inc., Palo Alto, CA 94304 (US)
(72) Inventor: MEDEMA, Ryan, Georgetown, 78633 (US); DOLL, Sean, Cedar Park, 78613 (US); CAMPO, Elizabeth, Austin, 78757 (US); ZHANG, Hongxuan, Austin, 78750 (US)
(74) Representative: Mathisen & Macara LLP

(57) **Abstract**

A cryoablation apparatus includes a Dewar (102) configured to retain a volume of cryogen (110) and a heater assembly positioned in the Dewar (102). The heater assembly includes at least one heater (120) configured to heat a targeted portion of the cryogen (110) as the volume of cryogen in the Dewar (120) changes. The targeted portion of the cryogen is located at or near a top surface of a liquid level L1 of the liquid cryogen.

## Description

### FIELD

The present disclosure relates to apparatuses and methods for targeted vaporization of cryogen. More particularly, the present disclosure relates to apparatuses and methods for the targeted vaporization of cryogen in a Dewar to pressurize the Dewar for efficient transfer of liquid cryogen for cryoablation procedures.

### BACKGROUND

This section provides background information related to the present disclosure which is not necessarily prior art.

Systems and methods for providing cryoablation treatments may include cryoablation probes that are introduced at or near target tissue in a patient. A cryoablation system may include an extremely cold cryogen (liquid, gas, or mixed phase Nitrogen, Argon, Helium, or the like) that may be passed through a probe that is in thermal contact with the target tissue. Heat from the tissue passes from the tissue, through the probe, and into the cryogen that removes heat from the targeted tissue. This removal of heat causes tissue to freeze, resulting in the destruction of the targeted tissue. When the tissue freezes, ice forms typically in an iceball. The iceball may be in the form a sphere, ellipsoid or other rounded shape. It is desirable to perform cryoablation treatments such that the target tissue is completely frozen and that the freezing of surrounding tissues and/or body structures is minimized.

Traditional or existing systems often include cryogen sources and delivery systems that operate to deliver the cryogen to the probe to create satisfactory and predictable temperatures at the target tissue. Existing systems, however, may include multiple separate systems, individual controls, independent measurement devices, and individual user interfaces that can lead to inefficiencies and treatments of reduced effectiveness. There exists a need, therefore, for improved apparatuses and systems for improved efficiency, lower treatment cost, and improved treatment effectiveness.

### SUMMARY

This section provides a general summary of the disclosure, and is not a comprehensive disclosure of its full scope or all of its features.

In some embodiments of the present disclosure, a cryoablation apparatus may include a container or Dewar configured to retain a volume of cryogen and a heater assembly positioned in the Dewar. The heater assembly may include at least one heater configured to heat a targeted portion of the cryogen as the volume of cryogen in the Dewar changes. The cryoablation apparatus may further include a transfer conduit, a pump assembly, and a supply conduit. The Dewar may be insulated and/or made of suitable materials to maintain the cryogen at low temperatures used for cryoablation treatments. The transfer conduit may extend into the liquid cryogen from the pump assembly. The transfer conduit may be a tube or other lumen that fluidly connects the internal volume of the Dewar to the pump assembly. The liquid cryogen may flow from the Dewar through the transfer conduit. The pump assembly may include a pump, a motor, and/or other elements to cause liquid cryogen to flow from the transfer conduit to the supply conduit. The supply conduit may be fluidly coupled to a manifold, valve, and/or other elements to provide the liquid cryogen to the from the Dewar to one or more cryoablation probes. The pump may be positioned inside the Dewar and/or coupled to other locations of the transfer conduit. In some examples, the Dewar may be of sufficient size to perform multiple cryoablation cycles. In some examples, the Dewar may be of suitable size to hold about 25 to about 50 liters of cryogen. The Dewar may be configured as a pressure vessel. In some embodiments, the cryoablation apparatus includes a pressure sensor, arranged to monitor the pressure inside the Dewar. In some embodiments the cryoablation apparatus include a device to monitor the level of liquid in the Dewar. The level of liquid in the Dewar may be monitored by a controller, computing device, PLC or other suitable device. The apparatus may include one or more liquid level sensors. Any suitable liquid level sensor may be used, for example impedance sensors. The one or more liquid level sensors may be included in each of the heaters. The level of liquid in the Dewar may be monitored by a continuous liquid level sensor. In some embodiments, the cryoablation apparatus further includes a dispenser, configured to dispense one or more pressure-inducing tablets. The dispenser may be located in any suitable location relative to the Dewar, including in the top or side wall of the Dewar. In some examples, the pressure-inducing tablets may be an effervescent tablet. In other examples, a different tablet or a chemical in a different form may be used to increase the pressure in the Dewar. Chemical tablet or similar material vaporization/heating/expansion effects for gas nitrogen pressurization may be achieved with different chemicals, including but not limited to tablet, gas, size/volume expansion characteristics. Various chemicals and materials can be utilized for this pressurization purpose to increase the Dewar pressure efficiency without adding any contamination or noise to the system and device

In one aspect, the targeted portion of the cryogen may include a portion of the cryogen at or near a liquid level of the cryogen.

In another aspect, the targeted portion of the cryogen may include a portion within a predetermined distance from the liquid level of the cryogen.

In another aspect, the heater may convert liquid cryogen to cryogen vapor.

In another aspect, the heater assembly may be connected to a transfer conduit inside the Dewar.

In another aspect, the heater assembly may be coupled to a wall of the Dewar. In some embodiments, the heater assembly may be positioned inside the Dewar and/or connected at or near a wall of the Dewar. In other embodiments the heating assembly is integrated into the walls of the Dewar.

In another aspect, the heater assembly may be coupled to a support bar in the Dewar. The support bar may be a structure positioned in the Dewar that is configured to support the heating assembly in a desired position in the Dewar. The support bar may be a stand-alone support structure. In other examples, the support bar may also provide other functionality in addition to supporting the heating assembly. For example, the support bar may be a tube that can be used to re-fill the Dewar. In still other examples, the support bar may provide other functionality.

In another aspect, the heater assembly may include a plurality of heaters. Each of the plurality of heaters may be positioned at a different height relative to a base of the Dewar. For example, the heating assembly may comprise 2, 4, 6, 8, 10 or more heaters. The heater of the heating assembly may comprise a heating element. The controller may energize the heating element and may control the power delivered including control of the power signal, amplitude, frequency, duty cycle, pulse width, or other characteristic to cause a desired amount of energy to be supplied to heat the liquid cryogen as desired. In other examples, the heater of the heating assembly may comprise any suitable heating means, such as an infrared heater, laser heater, power or energy conversion heaters, such as wireless power, emission power, radio frequency (RF) or microwave heaters, pressure heaters, or others which are able to transmit heat or power to the Dewar and achieve vaporization of the liquid cryogen.

In another aspect, the plurality of heaters may be positioned inside an insulated enclosure in the Dewar. For example, the cryoablation apparatus may also include a sleeve. The sleeve may be positioned around the heating assembly. The sleeve may, for example, have a cylindrical shape and be positioned radially outward of the heating assembly. The sleeve may have an outer layer of insulation. The sleeve may, for example, include a vacuum chamber positioned between an inner and outer wall of the sleeve.

In another aspect, the at least one heater may be positioned at a top of the Dewar. The heater, in this example, may be configured as a radiation heat element that may heat a targeted portion of the liquid cryogen via radiation. In some embodiments the Dewar may include a radiative heat shield positioned on an inner wall of the Dewar and/or on an inner side of the lid or top of the Dewar to direct heat toward the liquid cryogen and reduce or limit the heating of the Dewar.

In another aspect, the at least one heater may be configured to move in the Dewar.

In another aspect, the at least one heater may be slidably positioned on a shaft in the Dewar and the at least one heater may be configured to slide along the shaft as a liquid level of the cryogen in the Dewar changes. For example, the cryoablation apparatus may include a heating assembly including an upper collar, a lower collar, a shaft, and a heater. The heater may be positioned on the shaft such that the heater may slide or move along an axial length of the shaft. The lower collar may be configured to prevent the heater from sliding off the shaft. The heater may comprise an opening shaped to accept a shaft, to allow the heater to slide along the axial length of the shaft in use. The opening may have a non-circular cross-sectional shape, which has the advantage of preventing the heater rotating around the longitudinal axis of the shaft in use. In other examples, the opening may have other cross-sectional shapes or the heater may include other anti-rotation features, such as a groove and tooth, or the like. In some embodiments the shaft may provide the transfer conduit. Alternatively, the shaft may be separate from the transfer conduit. In yet other examples, the shaft may be positioned in a different position and may be connected to a top or base of the Dewar.

In another aspect, the at least one heater comprises a float having a predetermined buoyancy that is configured to maintain the at least one heater at a predetermined position relative to the liquid level of the cryogen in the Dewar. The float may be attached to the heater. The float may be above or below the heating element of the heater, in use. The float may have any suitable shape. The heater may comprise more than one heating element. A platform may be present to separate the float from the heating element of the heater. The heater may automatically adjust position or move as the liquid level changes in the Dewar. Various suitable materials may be used for the float such as a suitable plastic, alloy, stainless steel, foam, or other material.

Various cryogens may be used such as helium, argon, nitrogen or the like. In another aspect, the cryogen may be Nitrogen.

In some embodiments of the present disclosure, a method of heating a targeted portion of cryogen is provided. The method may include obtaining cryogen liquid level information characterizing a location of a liquid level of cryogen in a Dewar, obtaining pressure information characterizing an internal pressure of the Dewar, and energizing a heater in the Dewar to heat a targeted portion of the cryogen in the Dewar when the pressure information indicates that the internal pressure of the Dewar is less than a predetermined pressure value.

In one aspect, the targeted portion of the cryogen may include a portion of the cryogen at or near the liquid level of the cryogen.

In another aspect, the targeted portion of the cryogen may include a portion within a predetermined distance from the liquid level of the cryogen.

In another aspect, the heater is a first heater and the method further includes de-energizing the first heater when the liquid level moves below the first heater and energizing a second heater, wherein the second heater is located at a predetermined distance below the first heater.

In another aspect, the method may also include de-energizing the heater when the pressure information indicates that the internal pressure of the Dewar is greater than a predetermined threshold.

In another aspect, the heater may be slidably positioned on a shaft in the Dewar and the heater is configured to slide along the shaft as the liquid level of the cryogen in the Dewar changes.

Further areas of applicability will become apparent from the description provided herein. The description and specific examples in this summary are intended for purposes of illustration only and are not intended to limit the scope of the present disclosure.

### DRAWINGS

The drawings described herein are for illustrative purposes only of selected embodiments and not all possible implementations, and are not intended to limit the scope of the present disclosure.
FIG. 1 is an illustration of an example cryoablation apparatus in accordance with some embodiments of the present disclosure.
FIG. 2 is an illustration of the cryoablation apparatus of FIG. 1 shown in with the liquid cryogen at a different level.
FIG. 3 is an illustration of another example cryoablation apparatus in accordance with some embodiments of the present disclosure.
FIG. 4 is an illustration of another example cryoablation apparatus in accordance with some embodiments of the present disclosure.
FIG. 5 is an illustration of another example cryoablation apparatus in accordance with some embodiments of the present disclosure.
FIG. 6 is an illustration of another example cryoablation apparatus in accordance with some embodiments of the present disclosure.
FIG. 7 is an illustration of aspects of a cryoablation apparatus that may include a moving heater assembly in accordance with some embodiments of the present disclosure.
FIG. 8 is an illustration of an example heater assembly that may be used in various cryoablation apparatuses of the present disclosure.
FIG. 9 is an illustration of another example cryoablation apparatus in accordance with some embodiments of the present disclosure.
FIG. 10 is a flow chart illustrating an example method of pressurizing a cryoablation apparatus in accordance with some embodiments of the present disclosure.

Corresponding reference numerals indicate corresponding parts throughout the several views of the drawings.

### DETAILED DESCRIPTION

Example embodiments will now be described more fully with reference to the accompanying drawings.

Example embodiments are provided so that this disclosure will be thorough, and will fully convey the scope to those who are skilled in the art. Numerous specific details are set forth such as examples of specific components, devices, and methods, to provide a thorough understanding of embodiments of the present disclosure. It will be apparent to those skilled in the art that specific details need not be employed, that example embodiments may be embodied in many different forms and that neither should be construed to limit the scope of the disclosure. In some example embodiments, well-known processes, well-known device structures, and well-known technologies are not described in detail.

The terminology used herein is for the purpose of describing particular example embodiments only and is not intended to be limiting. As used herein, the singular forms "a," "an," and "the" may be intended to include the plural forms as well, unless the context clearly indicates otherwise. The terms "comprises," "comprising," "including," and "having," are inclusive and therefore specify the presence of stated features, integers, steps, operations, elements, and/or components, but do not preclude the presence or addition of one or more other features, integers, steps, operations, elements, components, and/or groups thereof. The method steps, processes, and operations described herein are not to be construed as necessarily requiring their performance in the particular order discussed or illustrated, unless specifically identified as an order of performance. It is also to be understood that additional or alternative steps may be employed.

When an element or layer is referred to as being "on," "engaged to," "connected to," or "coupled to" another element or layer, it may be directly on, engaged, connected or coupled to the other element or layer, or intervening elements or layers may be present. In contrast, when an element is referred to as being "directly on," "directly engaged to," "directly connected to," or "directly coupled to" another element or layer, there may be no intervening elements or layers present. Other words used to describe the relationship between elements should be interpreted in a like fashion (e.g., "between" versus "directly between," "adjacent" versus "directly adjacent," etc.). As used herein, the term "and/or" includes any and all combinations of one or more of the associated listed items.

Although the terms first, second, third, etc. may be used herein to describe various elements, components, regions, layers and/or sections, these elements, components, regions, layers and/or sections should not be limited by these terms. These terms may be only used to distinguish one element, component, region, layer or section from another region, layer or section. Terms such as "first," "second," and other numerical terms when used herein do not imply a sequence or order unless clearly indicated by the context. Thus, a first element, component, region, layer or section discussed below could be termed a second element, component, region, layer or section without departing from the teachings of the example embodiments.

Spatially relative terms, such as "inner," "outer," "beneath," "below," "lower," "above," "upper," and the like, may be used herein for ease of description to describe one element or feature's relationship to another element(s) or feature(s) as illustrated in the figures. Spatially relative terms may be intended to encompass different orientations of the device in use or operation in addition to the orientation depicted in the figures. For example, if the device in the figures is turned over, elements described as "below" or "beneath" other elements or features would then be oriented "above" the other elements or features. Thus, the example term "below" can encompass both an orientation of above and below. The device may be otherwise oriented (rotated 90 degrees or at other orientations) and the spatially relative descriptors used herein interpreted accordingly.

The present disclosure is directed to apparatuses and methods for delivering liquid cryogen to a cryoablation probe. The methods and apparatuses may deliver liquid cryogen to a cryoablation probe during a cryoablation treatment in some examples. The methods and apparatuses of the present disclosure are improvements over known or existing apparatuses and methods. The methods and apparatuses of the present disclosure may operate to deliver liquid in a more efficient manner, in a shorter period of time, with less cost, and with less energy consumption over existing apparatuses and methods.

In various embodiments as further described below, the apparatuses and methods of the present disclosure include heating assemblies positioned inside a Dewar that are configured to heat a targeted portion of the cryogen. The heating of the targeted portion of the cryogen causes some of the cryogen to change from liquid cryogen to a cryogen vapor. The cryogen vapor is less dense than the liquid cryogen and may cause the internal pressure of the Dewar to increase. The increased internal pressure in the Dewar may assist with moving the liquid cryogen from the Dewar through one or more transfer conduits to a cryoprobe or other elements.

The apparatuses and methods of the present disclosure are improvements over existing or known systems in that the heating assemblies heat a targeted portion of the cryogen at or near a liquid level of the cryogen in the Dewar. This targeting of a portion of the liquid cryogen at the liquid level (e.g., at or near the top surface of the liquid cryogen) enables the formed cryogen vapor to exit the volume of liquid cryogen and fill the top portion of the Dewar above the liquid cryogen. This targeting allows reduced amounts of energy to be used to pressurize the Dewar because the vaporized cryogen does not need to travel through a significant distance through the liquid cryogen where is may change back into liquid cryogen before exiting the liquid cryogen and filling the top portion of the Dewar. Furthermore, the temperature of the liquid cryogen can be maintained at a lower overall temperature than if vaporized cryogen travels through the liquid cryogen.

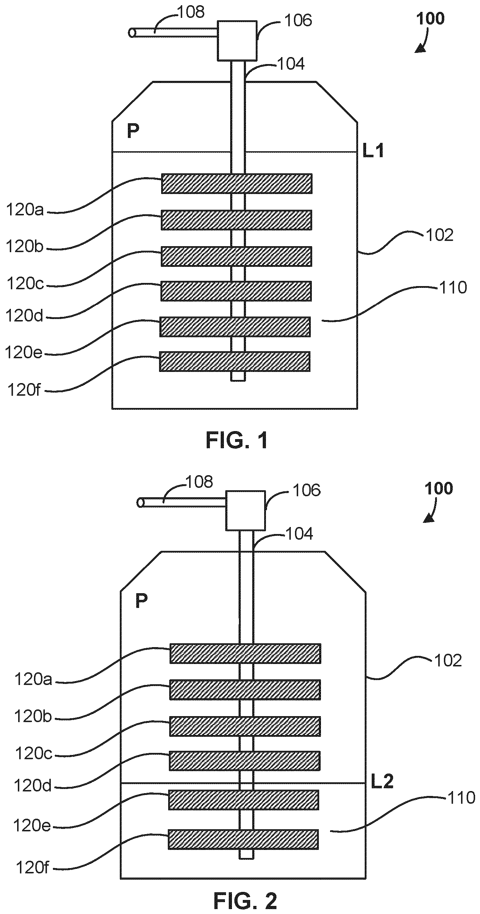

Referring now to FIGs. 1 and 2, an example cryoablation apparatus 100 is shown. The cryoablation apparatus 100 may be used, for example, to deliver a liquid cryogen to one or more cryoablation probes (not shown). The cryoablation apparatus 100 may include a container or Dewar 102, a transfer conduit 104, a pump assembly 106, and a supply conduit 108. The Dewar 102 may be a container that is used to retain a volume of cryogen. The Dewar 102 may be insulated and/or made of suitable materials to maintain the cryogen at low temperatures used for cryoablation treatments. In some examples, the Dewar may be of sufficient size to perform multiple cryoablation cycles. In some examples, the Dewar 102 may be of suitable size to hold about 25 to about 50 liters of cryogen. Various cryogens may be used such as Helium, Argon, Nitrogen or the like. In a preferred example, liquid Nitrogen may be used as the cryogen for the cryoablation treatments.

The Dewar 102 may be filled with a volume of cryogen. The volume of the cryogen may position the liquid level L1 of the cryogen at a position at or near a top of the Dewar 102 when the Dewar 102 is in a filled or near-filled condition as shown in FIG. 1. After multiple cryoablation cycles are performed, the cryogen may be depleted such that the liquid level L1 drops from the filled condition to a depleted liquid level L2 as shown in FIG. 2. When the cryogen reaches a predetermined threshold, the Dewar 102 may need to be re-filled to return the Dewar 102 to the filled condition as illustrated in FIG. 1.

The transfer conduit 104 may extend into the liquid cryogen 110 from the pump assembly 106. The transfer conduit 104 may be a tube or other lumen that fluidly connects the internal volume of the Dewar 102 to the pump assembly 106. The liquid cryogen may flow from the Dewar 102 through the transfer conduit 104. The pump assembly 106 may include a pump, a motor, and/or other elements to cause liquid cryogen to flow from the transfer conduit 104 to the supply conduit 108. The supply conduit 108 may be fluidly coupled to a manifold, valve, and/or other elements to provide the liquid cryogen to the from the Dewar 102 to one or more cryoablation probes.

In other examples, the cryoablation apparatus 100 may include other elements that may not be shown in FIGs. 1 and 2 and/or may include alternate configurations. In other examples, the cryoablation apparatus 100 may include a pump positioned inside the Dewar and/or coupled to other locations of the transfer conduit 104. As can be appreciated, the features of the present disclosure may be included on such alternate configurations of the cryoablation apparatus 100. The description below that describes various elements used in connection with the cryoablation apparatus 100 for illustration purposes and should not be interpreted as limiting the use of such elements.

The cryoablation apparatus 100 also includes a plurality of heaters 120a to 120f. The heaters 120 are positioned and distributed along the transfer conduit 104 in the Dewar 102. Each of the heaters 120a to 120f may be positioned in at a different vertical height in the Dewar 102. In the example shown, the heater array includes six heaters 120a to 120f. It should be appreciated, however, that the cryoablation apparatus 100 may include more than six heaters 120 or less than six heaters 120. The heaters 120 may be positioned at different vertical heights in the Dewar so that a heater may be energized to heat a targeted portion of the cryogen 110 that is located at or near a top surface of the liquid cryogen. The heater 120 that is energized may correspond to a heater 120 that is located at a predetermined range from the liquid level L1. The heater that is energized may correspond to a heater 120 that is located closest to the liquid level L1 while still being submerged in the liquid cryogen.

Each of the heaters 120a to 120f in the heater array may include a suitable heating element to heat the liquid cryogen 110 positioned at or near the heater. The heating element, for example, may be a resistive heating element coupled to a suitable power source and controller. The controller may energize the heating element and may control the power delivered including control of the power signal, amplitude, frequency, duty cycle, pulse width, or other characteristic to cause a desired amount of energy to be supplied to heat the liquid cryogen as desired. In other examples, the heaters 120 and/or the included heating element may have other configurations such as an infrared heater, or laser heater. The heaters may also include power or energy conversion heaters, such as wireless power, emission power, radio frequency (RF) or microwave heaters, pressure heaters, or others which are able to transmit heat or power to the Dewar and achieve vaporization of the liquid cryogen.

Each of the heaters 120a to 120f may be energized as desired to heat liquid cryogen 110 to cause the liquid cryogen 1110 to be vaporized to cryogen gas. The cryogen gas may rise and occupy a top portion of the Dewar 102 that may have a pressure P as shown in FIG. 1. As more liquid cryogen is heated and changed to cryogen gas, the pressure P in the Dewar may increase. The pressure P may exert a force on the liquid cryogen 110. This force may urge the liquid cryogen into the transfer conduit 104. This force may also urge the liquid cryogen 110 into a pump located in the cryoablation apparatus 100. Thus, the creation of the cryogen gas from the liquid cryogen 110 may prime the pump of the cryoablation apparatus 100. The pressure required to prime the pump of the cryoablation apparatus 100 may have a predetermined pressure range or predetermined pressure threshold. For example, it may be desirable to heat enough liquid cryogen to change the liquid cryogen to cryogen gas and pressurize the Dewar to an internal pressure in a range of about 10 psi to about 15 psi. In other examples, a predetermined pressure threshold of about 10 psi may be used. In still other examples, other pressure ranges may be used which may depend on the pump design, pressure tolerance, heater efficiency, and/or other factors. For example, if the Dewar were to be configured as a pressure vessel, pressure ranges or pressure thresholds in excess of 15 psi could be used.

The pressurization of the Dewar to the predetermined pressure threshold or predetermined pressure range also serves an additional purpose. The increased pressure in the Dewar may raise the boiling point of the liquid cryogen (e.g. liquid nitrogen) in the Dewar. This raised boiling point may assist in preventing cryogen gas from entering the pump. It is desirable to prevent cryogen gas from entering the pump because the cryogen gas may generate heat when pressurized and inhibit the proper functioning of the pump.

During a cryoablation cycle, the liquid cryogen 110 may be moved from the Dewar 102 to a cryoablation probe. The cryogen may be returned to the Dewar and/or exhausted to ambient. The cryogen may be depleted during cryoablation cycles such that the liquid level L1 of the liquid cryogen 110 moves downward in the Dewar 102. Such a condition is shown in FIG. 2. The different heaters 102a to 102f may be alternately and/or successively energized during treatments so that a desired heater is heating a targeted portion of the liquid cryogen. In some examples, one of the heaters 102a to 102f is energized to heat a top portion of the liquid cryogen located at or near the liquid level L1.

For example, when a cryoablation treatment is started, the heater 102a may be energized. Since the heater 102a is located at or near the liquid level L1 only a targeted portion of the liquid cryogen is heater at or near the top surface of the liquid cryogen 110. The cryogen gas that is created from such heating only needs to travel a short vertical distance before entering the top portion of the Dewar 102 and causing the pressure P to increase in the Dewar. A controller, computing device, PLC, or other device may be used to monitor the location of the liquid level L1. In some examples, one or more liquid level sensors may be included in each of the heaters 102a to 102f. In other examples, the cryoablation apparatus 100 may include a series of liquid level sensors at other locations or include continuous liquid level sensor. An impedance may be collected, for example, to determine a position of the liquid level L1 in the Dewar 102. The controller may then energize the heater 102a to 102f that is located closest to the liquid level L1 and below the top surface of the liquid cryogen 110.

As the liquid level L1 drops in the Dewar 102, the controller may de-energize one heater and energize a different heater that is located at or below the liquid level L1. In this manner, the controller may successively de-energize the heaters 102a to 102f vertically as the liquid level L1 drops in the Dewar. As shown in FIG. 1, heater 102a is located closest to the liquid level L1 and below the surface of the liquid cryogen 110. This may be the first heater energized during operation of the cryoablation apparatus 100. As shown in FIG. 2, the liquid level L2 has dropped in the Dewar 102. In this circumstance, the controller may energize the heater 120e since it is located closest to the liquid level L2 and below a top surface of the liquid cryogen 110.

The heating array or heating assembly that includes multiple heaters, such as the heaters 102a to 102f, is an improvement over existing or known apparatuses. As discussed above, by targeting a portion of the cryogen at or near the liquid level of the liquid cryogen, the cryogen gas only needs to travel a short vertical distance before occupying the top portion of the Dewar 102 located above the liquid cryogen 110. The cryogen gas is less likely to be cooled enough to cause the cryogen gas to change back to liquid cryogen. Thus, the creation the cryogen gas is more efficiently performed and less energy is required to pressurize the Dewar 102. Additionally, the temperature of the liquid cryogen is not unnecessarily increased. In apparatuses that include heaters that are located at or near the base of the Dewar 102 or at a distal end of the transfer conduit 104, the liquid cryogen may be heated at a position well below the top surface of the liquid cryogen. This may raise the overall temperature of the liquid cryogen. By targeting a top portion of the liquid cryogen, the overall temperature of the liquid cryogen may be maintained at a lower temperature that then can be used to perform the cryoablation treatment. Observations and testing has demonstrated that the apparatuses and methods of the present disclosure may allow the liquid cryogen to be maintained and used at a temperature of about -196°C whereas existing apparatuses and methods may only be able to maintain and use liquid cryogen at a temperature of about-185°C.

Existing systems and methods also suffer from long delays before liquid cryogen is ready to flow for a cryogen treatment. As discussed above, a cryoablation cycle may not be performed until the internal pressure in the Dewar 102 is increased to a predetermined threshold or to a predetermined range. Existing systems that heat liquid cryogen using other heating arrangements or systems (e.g. heaters positioned well below the liquid level of the liquid cryogen) need increased energy and significant time until the internal pressure of the Dewar 102 reaches the predetermined pressure threshold or range. Existing systems may take times in excess of 1 hour to achieve suitable pressures and to begin a flow of cryogen. The cryoablation apparatus 100 and other apparatuses of the present disclosure can begin to move liquid cryogen more quickly than existing systems. The cryoablation apparatus 100, for example, may begin to move liquid cryogen in a period of about 3 minutes to about 5 minutes in embodiments with a resistive heater that may achieve a pressure of about 10 psi to about 15 psi at power levels in a range of about 200 watts to about 250 watts. In one example, in a Dewar that includes 20 liters (L) of liquid nitrogen, the time to achieve the predetermined pressure range was cut in half as compared to an existing apparatus that includes a fully immersed heater. In other examples, other types of heaters and with other configurations of Dewars or other factors, the time required to reach suitable pressures and/or to begin the flow of cryogen may take other periods of time.

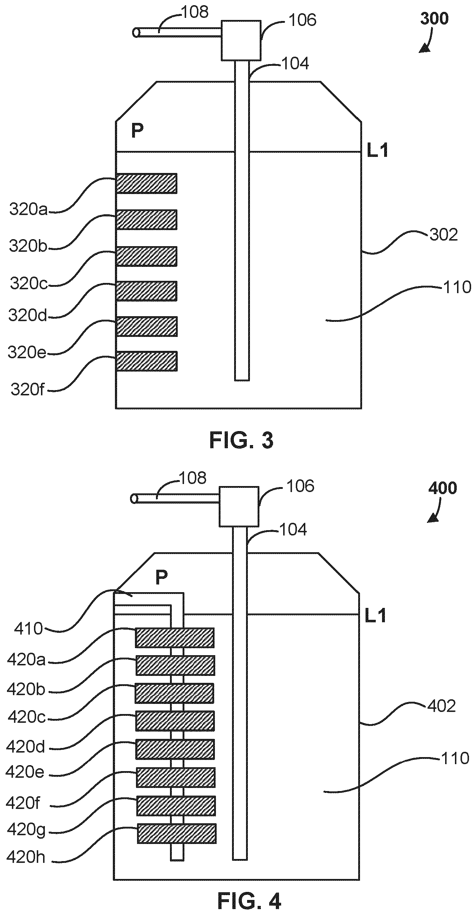

Referring now to FIG. 3, another example cryoablation apparatus 300 is shown. In this embodiment, the cryoablation apparatus 300 may be similar to the cryoablation apparatus 100 previously described. The cryoablation apparatus 300 may include the Dewar 302, the transfer conduit 104, the pump assembly 106, and the supply conduit 108. The cryoablation apparatus 300 may also include an array of heaters 320a to 320f. In this example, the heaters 320a to 320f may be positioned in the Dewar 302 but may be positioned and/or connected at or near a wall of the Dewar 302. The cryoablation apparatus 300 may operate to heat a targeted portion of the liquid cryogen 110 at or near the liquid level L1. The cryoablation apparatus 300 may be operated similarly to the cryoablation apparatus 100 and may provide the same or similar improvements and advantages as the cryoablation apparatus 100.

The heaters 320 may, in other examples, be integrated into the walls of the Dewar. For example, a resistive wire may be implanted, attached, or otherwise integrated into the wall of the Dewar 302. The wall of the Dewar 302 may include a reflective surface or property to minimize or reduce radiative energy transfer to the Dewar wall. The integrated heaters 320 may be arranged at different vertical locations and operated as previously described. In the example shown, the cryoablation apparatus 300 includes six heaters 320 but it may include other numbers of heaters 320, including more than six heaters 320 or less than six heaters 320. In still other examples, the heaters 320 may be arranged differently than as shown and may be connected to the Dewar at other locations, in a non-linear arrangement or in other configurations.

Referring now to FIG. 4, another example cryoablation apparatus 400 is shown. In this embodiment, the cryoablation apparatus 400 may be similar to the cryoablation apparatus 100, 300 previously described. The cryoablation apparatus 400 may include the Dewar 402, the transfer conduit 104, the pump assembly 106, and the supply conduit 108. The cryoablation apparatus 400 may also include an array of heaters 420a to 420h. The cryoablation apparatus 400 may also include a support bar 410. The support bar 410 may be a structure positioned in the Dewar 402 that is configured to support the heaters 420 in a desired position in the Dewar 402. The support bar 410 may be a stand-alone support structure that provides support for the array of heaters 420. In other examples, the support bar 410 may also provide other functionality in addition to supporting the array of heaters 420. For example, the support bar 410 may be a tube that can be used to re-fill the Dewar 402. In still other examples, the support bar 410 may provide other functionality.

The cryoablation apparatus 400 may operate to heat a targeted portion of the liquid cryogen 110 at or near the liquid level L1. The cryoablation apparatus 400 may be operated similarly to the cryoablation apparatus 100 or 300 and may provide the same or similar improvements and advantages as the cryoablation apparatus 100. The cryoablation apparatus 400 may include or be coupled to a controller (e.g., PLC, computing device, etc.) that may energize and de-energize the heaters 420 in a desired sequence to heat the targeted portion of the liquid cryogen 110. In the example shown, the cryoablation apparatus 400 includes eight heaters 420a to 420h but it may include other numbers of heaters 420, including more than eight heaters 320 or less than eight heaters 320. In still other examples, the heaters 420 may be arranged differently than as shown and may be arranged in a non-linear arrangement or in other configurations.

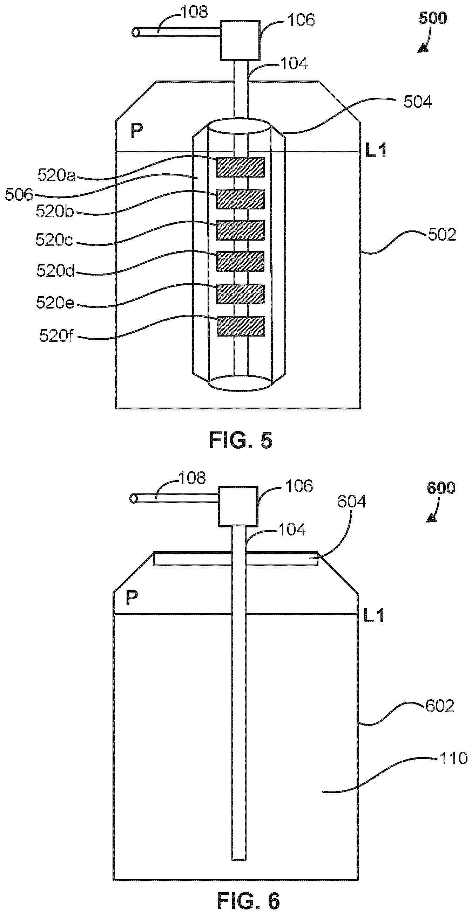

Referring now to FIG. 5, another example cryoablation apparatus 500 is shown. In this embodiment, the cryoablation apparatus 500 may be similar to the cryoablation apparatus 100, 300 previously described. The cryoablation apparatus 500 may include the Dewar 502, the transfer conduit 104, the pump assembly 106, and the supply conduit 108. The cryoablation apparatus 500 may also include an array of heaters 520a to 520f. The heaters 520a to 520f may be positioned on the transfer conduit 104 similarly to cryoablation apparatus 100.

The cryoablation apparatus 500 may also include sleeve 504. The sleeve 504 may be positioned around the array of heaters 520. The sleeve 504 may, for example, have a cylindrical shape and be positioned radially outward of the heaters 520. The sleeve 504 may have an outer layer of insulation (506) to separate the heaters 520 from the bulk liquid cryogen in the Dewar 502. The sleeve 504 may, for example, include a vacuum chamber positioned between an inner and outer wall of the sleeve 504. The vacuum chamber may insulate and may prevent or reduce thermal energy transfer from the liquid cryogen inside the sleeve 504 to the bulk liquid cryogen surrounding the sleeve 504. The sleeve may further isolate the targeted liquid cryogen that is heated by the heaters 520a to 520f when the heaters are energized during operation of the cryoablation apparatus 500. The sleeve 504 may also prevent the heating of the bulk liquid cryogen and assist in maintaining a lower overall temperature of the liquid cryogen than may be otherwise achieved.

The sleeve 504 may be included in the other cryoablation apparatuses of the present disclosure. The sleeve 504 may be positioned around the other heaters or the other heater arrays that may be included in the various embodiments of the cryoablation apparatuses described herein.

Referring now to FIG. 6, another example cryoablation apparatus 600 is shown. In this embodiment, the cryoablation apparatus 600 may be similar to the cryoablation apparatuses 100, 300 previously described. The cryoablation apparatus 600 may include the Dewar 602, the transfer conduit 104, the pump assembly 106, and the supply conduit 108. The cryoablation apparatus 600, in this example, may include a heater 604 positioned at or near a top of the Dewar 602. The heater 604, in this example, may be configured as a radiation heat element that may heat a targeted portion of the liquid cryogen 110 via radiation. The Dewar 602 may include a radiative heat shield positioned on an inner wall of the Dewar 602 and/or on an inner side of the lid or top of the Dewar 602 to direct heat toward the liquid cryogen and reduce or limit the heating of the Dewar 602.

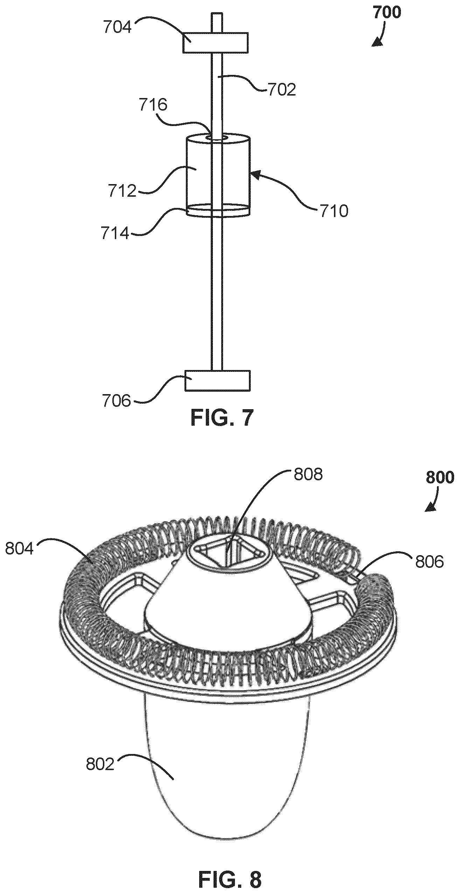

Referring now to FIG. 7, a heating assembly 700 is shown. The heating assembly 700 may be provided in the cryoablation apparatus 100, for example. Instead of including the array of heaters 120, the cryoablation apparatus 100 may include the heating assembly 700. The heating assembly 700 may include an upper collar 704, a lower collar 706, a shaft 702, and a heater 710. The heater 710 may be positioned on the shaft 702 such that the heater 710 may slide or move along an axial length of the shaft 702. An opening 716 may be provided in the heater 710 to allow the heater 710 to slide along the shaft 702.

The heating assembly 700 may be positioned in the Dewar 102. In some examples, the shaft 702 may operate as the transfer conduit 104 in addition to supporting the heating assembly 700. In other examples, the shaft 702 may be independent of the transfer conduit 104 and be positioned in the Dewar similarly to the support bar 410 shown in cryoablation apparatus 400. In yet other examples, the shaft 702 may be positioned in a different position and may be connected to a top or base of the Dewar 102.

The heating assembly 700 may include a float 712 and a heating element 714. The float 712 may provide buoyancy to the heater 710 and cause the heater 710 to float in a desired position relative to a liquid level L1 of the liquid cryogen 110. The buoyancy of the float 712 may also cause the heating element 714 to be positioned in a desired position relative to the liquid level L1 of the liquid cryogen 110. The float 712 may cause the heating element 714 to be positioned below a top surface of the liquid cryogen. The heating element 714 may be positioned so that the heating element is submerged below the top surface of the liquid cryogen 110. Various suitable materials may be used for the float 712 such as a suitable plastic, alloy, stainless steel, foam, or other material. The heating element 714 may include a resistive heating element or other suitable heating device as previously described with respect to heaters 120.

As can be appreciated, the heater 710 may slide along the shaft 702 as the liquid level L1 of the liquid cryogen changes during operating of the cryoablation apparatus. The float may keep the heating element in a desired position to heat a targeted portion of the liquid cryogen. In this manner, the heating assembly 700 may provide the improvement and advantages described above since a portion of the liquid cryogen is heated at or near a top surface of the liquid cryogen. In addition, the heater automatically adjusts or moves as the liquid level L1 changes in the Dewar 102. The top collar 704 may have an outer shape or size that limits movement of the heater 710 along the shaft 702. The lower or bottom collar 706 may also have an outer shape or size that limits movement of the heater 710 along the shaft 702. In addition, the lower collar 706 may prevent the heater from sliding off the shaft 702.

Referring now to FIG. 8, another example heater 800 is shown. The heater 800 have similar functionality and operate similarly to the heater 710 previously described. While not shown, the heater 800 may be positioned in the Dewar 102 and may be configured to slide along a shaft (not shown). In this example, the heater 800 includes a heating element 804, a float 802, and a platform 806. The heating element 804 may be a resistive heater such as coil of resistive wire through which a current is passed to generate heat. The resistive heater may have a circular or other suitable shape. The platform 806 may be similarly shaped as the resistive heater and be configured to support the resistive heater. The platform 806 in this example includes several openings to all the liquid cryogen to pass as the heater 800 moves along a cooperative shaft in the Dewar.

The float 802 in this example is positioned below the resistive heater. The combined buoyancy of the float 802, the platform 806, and the heating element 804 may be configured so that the heating element 804 is positioned below a top surface of the liquid cryogen in the Dewar. The heater 800 may include opening 808 that is sized and shaped to accept a shaft and allow the heater 800 to slide along the axial length thereof. In this example, the opening 808 has a non-circular shape. Such a configuration prevents the heater 800 from rotating around the shaft. Such an anti-rotation feature may be desirable to prevent wires or other connecting elements from wrapping around the shaft during operation that may limit movement of the heater 800. In other examples, the opening 808 may have other shapes or the heater 800 may include other anti-rotation features such as a groove and tooth, other non-circular shapes, or the like.

The heater 800 is but one embodiment of possible heaters that may be used. In other embodiments, the float 802 may have different shapes and the heating element 804 and/or the platform 806 may have different shapes and relative sizes. In yet other examples, the heater 800 may include more than one heating element 804 and the float 802 may be positioned above the heating element 804.

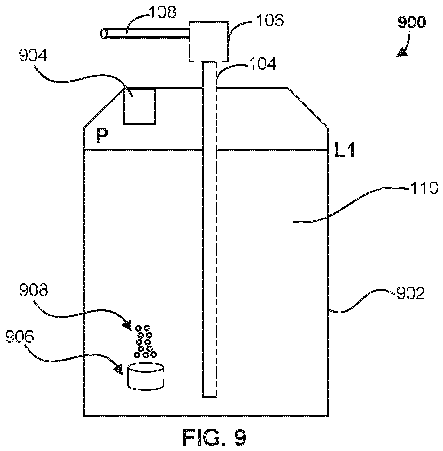

Referring now to FIG. 9, another example cryoablation apparatus 900 is shown. In this example, the cryoablation apparatus 900 operates to increase the internal pressure in the Dewar 902 when desired. The cryoablation apparatus 900, in this example, includes a dispenser 904. The dispenser 904 may include one or more pressure-inducing tablets 906. When it is desired to increase the pressure inside the Dewar 902, an operator or controller may cause the dispenser 904 to dispense one or more pressure-inducing tablets 906. The pressure-inducing tablets 906 may react with the liquid cryogen 110 to cause gas 908 to be generated that increases the internal pressure in the Dewar. The pressure-inducing tablets 906 may operate or react so as not to increase the overall temperature of the bulk liquid cryogen. Thus, the cryoablation apparatus 900 may provide the improvements and advantages over existing systems that was previously described. In some examples, the pressure-inducing tablets may be an effervescent tablet. In other examples, a different tablet or a chemical in a different form may be used to increase the pressure in the Dewar 902. Chemical tablet or similar material vaporization/heating/expansion effects for gas nitrogen pressurization may be achieved with different chemicals, including but not limited to tablet, gas, size/volume expansion characteristics, etc. Various chemicals and materials can be utilized for this pressurization purpose to increase the Dewar pressure efficiency may be applicable without adding any contamination or noise to the system and device.

In the example shown, the dispenser 904 is located at a top of the Dewar 902. In other examples, the dispenser 904 may be positioned in a wall of the Dewar or at other suitable location. The dispenser 904 may be a housing with an actuator that causes the tablet or other chemical to be introduced into the Dewar 902. In other examples, other dispensers, spouts, valves or other devices may be used to introduce the chemical or material into the Dewar 902.

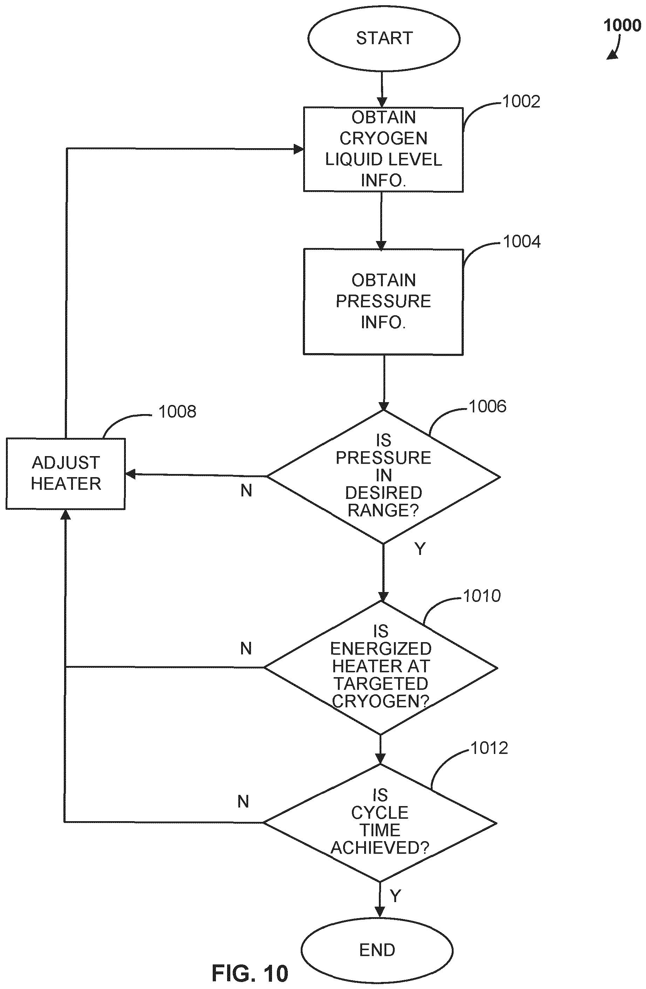

Referring now to FIG. 10, an example method 1000 is illustrated. The method 1000 may provide a method of heating a target portion of liquid cryogen. The method 1000 may be performed using one or more of the cryoablation apparatuses 100, 300, 400, 500, and/or 600 previously described. The method 1000 is described below with reference to the cryoablation apparatus 100 for illustration purposes only. It should be appreciated that the method 1000 may be performed by other cryoablation apparatuses of the present disclosure or variations thereof.

The method 1000 may begin at step 1002. At step 1002, the cryoablation apparatus 100 may obtain cryogen liquid level information. It should be appreciated that although not shown in FIG. 1, the cryoablation apparatus 100 or other cryoablation apparatuses of the present disclosure may include a cryoablation controller, computing device, application-specific circuit, PLC, or other controller that may perform one or more functions in combination with the elements shown in FIG. 1. For the sake of brevity, the term controller is used in describing the functions of method 1000 but it should be appreciated that use of the term controller does not limit the functions from being performed by other or multiple control devices such as those listed above.

The cryoablation apparatus 100 may obtain the cryogen liquid level information from one or more sensors (such as impedance sensors) that may be located in the Dewar 102. The cryogen liquid level information may characterize a position of the liquid level L1 in the Dewar 102.

At step 1004, the cryoablation apparatus 100 may obtain internal pressure information. The pressure information may be obtained from one or more pressure sensors positioned in the Dewar or in or at various positions of the cryogen flow path in the Dewar, or external to the Dewar. The pressure information may characterize a pressure in the cryogen flow path and/or in the Dewar 102.

At step 1006, the cryoablation apparatus 100 may determine whether the pressure information indicates if the pressure in the Dewar 102 is in a desired range. In order to initiate a cryoablation treatment, the internal pressure in the Dewar 102 may need to be in a desired range in order to prime the cryogen pump and/or to cause liquid cryogen to begin to flow from the Dewar 102 to the cryoablation probe. The desired range may be a predetermined pressure range to cause such action to occur. In some examples, the predetermined or desired pressure range may be a range of about 10 psi to about 15 psi. In other examples, other pressure ranges may be used. If the pressure information indicates that the pressure is in the desired range, further action may not be required and the method 1000 may proceed to step 1010. If the pressure information indicates that the pressure in the Dewar 102 is not in the desired range, the method 1000 may proceed to step 1008.

At step 1008, the controller may adjust the heater. The heater may be any one or more of the various heaters and heating assemblies described above. In one example, the controller may adjust the heater based on the cryogen liquid level information obtained at step 1002. The controller may determine which heater of an array of heaters is located at or near a targeted portion of the liquid cryogen. The targeted portion may be at or near a surface or liquid level L1 of the liquid cryogen. The controller may then energize a heater located at and/or below the liquid level L1 of the liquid cryogen. The controller may cause a power signal with desired characteristics (e.g., voltage, current, pulse width, duty cycle, etc.) to be delivered to an identified heater. The heater may then heat the targeted cryogen to change liquid cryogen to cryogen gas that may cause the pressure in the Dewar 102 to increase. The method 1000 may then return to step 1002 where the liquid level information and the pressure information is again obtained to determine if adjustments are needed.

At step 1010, the controller may determine whether an energized heater is located at the targeted cryogen. In this step 1010, the controller may consider the liquid level information to determine whether the correct heater is being energized. During a cryoablation treatment, the liquid cryogen may be used such that the liquid level drops below an energized heater. The controller may determine this and then de-energize one heater and energize the next heater in the heater array. The controller may take such action to cause a heater that is located closest to the liquid level and below the liquid level to be energized. Thus, efficient heating of the liquid cryogen is performed as the liquid cryogen is depleted and the liquid level moves downward in the Dewar 102. If the controller determines that the energized heater is located at the target cryogen, the method proceeds to step 1012. If the controller determines that the energized heater is not at the targeted cryogen (or is above the liquid level), the method moves to step 1008. At step 1008, the controller may take action as previously described to de-energize one heater and energize a successive heater located below the previously energized heater.

At step 1012, the controller may determine whether a cycle time has been achieved. Cryoablation cycles often have predetermined parameters such a time required to achieve a certain iceball size. The cycle time may also be determined by measuring temperatures at or near the cryoablation probe and/or monitoring a growth or size of the iceball. When the cycle time for the cryoablation cycle is achieved, the method may end. If the cycle time has not been achieved, the method may return to step 1008 whereby the heater is adjusted (if necessary). The method 1000 may then return to step 1002 whereby the steps are re-performed to monitor the cryogen liquid level, the internal pressure, and the cycle time to take action, if necessary, to adjust which heater is de-energized, or energized, and/or to adjust a power signal provided to the energized heater. In this manner, a closed-loop feedback loop is created to ensure that liquid cryogen flows to the cryogen probe in an efficient manner during operation of the cryoablation apparatus.

The foregoing description of the embodiments has been provided for purposes of illustration and description. It is not intended to be exhaustive or to limit the disclosure. Individual elements or features of a particular embodiment are generally not limited to that particular embodiment, but, where applicable, are interchangeable and can be used in a selected embodiment, even if not specifically shown or described. The same may also be varied in many ways. Such variations are not to be regarded as a departure from the disclosure, and all such modifications are intended to be included within the scope of the disclosure.

The following is a list of non-limiting illustrative embodiments disclosed herein:
Illustrative embodiment 1: A cryoablation apparatus comprising: a Dewar configured to retain a volume of cryogen; and a heater assembly positioned in the Dewar, the heater assembly comprising at least one heater configured to heat a targeted portion of the cryogen as the volume of cryogen in the Dewar changes.
Illustrative embodiment 2: The cryoablation apparatus of illustrative embodiment 1, wherein the targeted portion of the cryogen comprises a portion of the cryogen at or near a liquid level of the cryogen.
Illustrative embodiment 3: The cryoablation apparatus of illustrative embodiment 1, wherein the targeted portion of the cryogen comprises a portion within a predetermined distance from the liquid level of the cryogen.
Illustrative embodiment 4: The cryoablation apparatus of any of the preceding illustrative embodiments, wherein the heater is configured to convert liquid cryogen to cryogen vapor.
Illustrative embodiment 5: The cryoablation apparatus of any of the preceding illustrative embodiments, wherein the heater assembly is coupled to a transfer conduit inside the Dewar.
Illustrative embodiment 6: The cryoablation apparatus of any of illustrative embodiments 1 to 4, wherein the heater assembly is coupled to a wall of the Dewar.
Illustrative embodiment 7: The cryoablation apparatus of any of illustrative embodiments 1 to 4, wherein the heater assembly is coupled to a support bar in the Dewar.
Illustrative embodiment 8: The cryoablation apparatus of any of the preceding illustrative embodiments, wherein the heater assembly comprises a plurality of heaters each positioned at a different height relative to a base of the Dewar.
Illustrative embodiment 9: The cryoablation apparatus of illustrative embodiment 8, wherein the plurality of heaters are positioned inside an insulated enclosure in the Dewar.
Illustrative embodiment 10: The cryoablation apparatus of any of illustrative embodiments 1 to 4, wherein the at least one heater is positioned at a top of the Dewar.
Illustrative embodiment 11: The cryoablation apparatus of any of illustrative embodiments 1 to 4, wherein the at least one heater is configured to move in the Dewar.
Illustrative embodiment 12: The cryoablation apparatus of illustrative embodiments 1 to 4, wherein the at least one heater is slidably positioned on a shaft in the Dewar, the at least one heater configured to slide along the shaft as a liquid level of the cryogen in the Dewar changes.
Illustrative embodiment 13: The cryoablation apparatus of illustrative embodiment 12, wherein the at least one heater comprises a float having a predetermined buoyancy that is configured to maintain the at least one heater at a predetermined position relative to the liquid level of the cryogen in the Dewar.
Illustrative embodiment 14: The cryoablation apparatus of any of the illustrative embodiments herein, wherein the cryogen is Nitrogen.
Illustrative embodiment 15: A method of heating a targeted portion of cryogen comprising obtaining cryogen liquid level information characterizing a location of a liquid level of cryogen in a Dewar, obtaining pressure information characterizing an internal pressure of the Dewar, and energizing a heater in the Dewar to heat a targeted portion of the cryogen in the Dewar when the pressure information indicates that the internal pressure of the Dewar is less than a predetermined pressure value.
Illustrative embodiment 16: The method of illustrative embodiment 15, wherein the targeted portion of the cryogen comprises a portion of the cryogen at or near the liquid level of the cryogen.
Illustrative embodiment 17: The method of illustrative embodiment 15, wherein the targeted portion of the cryogen comprises a portion within a predetermined distance from the liquid level of the cryogen.
Illustrative embodiment 18: The method of illustrative embodiment 15, wherein the heater is a first heater and the method further comprises de-energizing the first heater when the liquid level moves below the first heater, and energizing a second heater, wherein the second heater is located at a predetermined distance below the first heater.
Illustrative embodiment 19: The method of illustrative embodiment 15, further comprising de-energizing the heater when the pressure information indicates that the internal pressure of the Dewar is greater than a predetermined threshold.
Illustrative embodiment 20: The method of illustrative embodiment 15, wherein the heater is slidably positioned on a shaft in the Dewar, the heater configured to slide along the shaft as the liquid level of the cryogen in the Dewar changes.

## Claims

1. A cryoablation apparatus(100, 300, 400, 500, 600, 700, 800, 900) comprising:
a Dewar (102, 302, 402, 502, 602, 902) configured to retain a volume of cryogen (110); and
a heater assembly positioned in the Dewar (102, 302, 402, 502, 602, 902), the heater assembly comprising at least one heater (120, 320, 420, 520, 604, 710, 804) configured to heat a targeted portion of the cryogen (110) as the volume of cryogen in the Dewar (102, 302, 402, 502, 602, 902) changes.

2. The cryoablation apparatus of claim 1, wherein the targeted portion of the cryogen comprises a portion of the cryogen at or near a liquid level (L1) of the cryogen (110), and optionally the targeted portion of the cryogen (110) comprises a portion within a predetermined distance from the liquid level (L1) of the cryogen.

3. The cryoablation apparatus of claim 1 or 2, wherein the heater (120, 320, 420, 520, 604, 710, 804) is configured to convert liquid cryogen (110) to cryogen vapor.

4. The cryoablation apparatus of claim 1, 2 or 3, wherein the heater assembly is coupled to a transfer conduit (104) inside the Dewar, and or a wall of the Dewar (102, 302, 402, 502, 602, 902), and/or a support bar (410) in the Dewar.

5. The cryoablation apparatus of any one of the preceding claims, wherein the heater assembly comprises a plurality of heaters (120, 320, 420, 520, 604, 710, 804) each positioned at a different height relative to a base of the Dewar (102, 302, 402, 502, 602, 902), optionally the plurality of heaters (120, 320, 420, 520, 604, 710, 804) are positioned inside an insulated enclosure (504, 506) in the Dewar (102, 302, 402, 502, 602, 902).

6. The cryoablation apparatus of any one of the preceding claims, wherein the at least one heater (120, 320, 420, 520, 604, 710, 804) is positioned at a top of the Dewar (102, 302, 402, 502, 602, 902), and optionally the at least one heater (120, 320, 420, 520, 604, 710, 804) is configured to move in the Dewar (102, 302, 402, 502, 602, 902).

7. The cryoablation apparatus of any one of the preceding claims, wherein the at least one heater (120, 320, 420, 520, 604, 710, 804) is slidably positioned on a shaft (104, 702) in the Dewar (102, 302, 402, 502, 602, 902), the at least one heater (120, 320, 420, 520, 604, 710, 804) configured to slide along the shaft (104, 702) as a liquid level of the cryogen in the Dewar changes.

8. The cryoablation apparatus of claim 7, wherein the at least one heater (120, 320, 420, 520, 604, 710, 804) comprises a float (802) having a predetermined buoyancy that is configured to maintain the at least one heater (804) at a predetermined position relative to the liquid level of the cryogen in the Dewar (102, 302, 402, 502, 602, 902).

9. The cryoablation apparatus of any one of the preceding claims, wherein the cryogen (110) is nitrogen.

10. A method of heating a targeted portion of cryogen (1000) comprising:
obtaining cryogen liquid level (1002) information characterizing a location of a liquid level of cryogen (110) in a Dewar (102, 302, 402, 502, 602, 902);
obtaining pressure information characterizing an internal pressure of the Dewar (1004); and
energizing a heater (120, 320, 420, 520, 604, 710, 804) in the Dewar (102, 302, 402, 502, 602, 902) to heat a targeted portion of the cryogen in the Dewar when the pressure information indicates that the internal pressure of the Dewar (102, 302, 402, 502, 602, 902) is less than a predetermined pressure value (1006).

11. The method of claim 10, wherein the targeted portion of the cryogen comprises a portion of the cryogen at or near the liquid level L1 of the cryogen (110).

12. The method of claim 10 or 11, wherein the targeted portion of the cryogen comprises a portion within a predetermined distance from the liquid level L1 of the cryogen(110).

13. The method of claim 10, 11 or 12, wherein the heater is a first heater and the method further comprises:
de-energizing the first heater when the liquid level moves below the first heater; and
energizing a second heater, wherein the second heater is located at a predetermined distance below the first heater.

14. The method of any one of claims 10-13, further comprising de-energizing the heater when the pressure information indicates that the internal pressure of the Dewar (102, 302, 402, 502, 602, 902) is greater than a predetermined threshold (1006).

15. The method of any one of claims 10-14, wherein the heater is slidably positioned on a shaft (104, 702, 410) in the Dewar (102, 302, 402, 502, 602, 902), the heater configured to slide along the shaft as the liquid level of the cryogen (110) in the Dewar changes.
